# EUROPEAN PATENT SPECIFICATION

(11) **EP 1 406 471 B1**
(45) Date of publication and mention of the grant of the patent: **30.08.2006**
(21) Application number: 03027427.8
(22) Date of filing: 07.07.2000
(51) Int. Cl.: H05B 1/02, A61M 5/44

(54) **Connector assembly for apparatus for regulating the temperature of a fluid**
Verbinderanordnung für Gerät zur Temperaturregelung einer Flüssigkeit
Ensemble connecteur pour appareil de régulation de la température d'un fluide

(30) Priority: 26.10.1999 US 426717; 26.10.1999 US 426752
(43) Date of publication of application: 07.04.2004
(62) Divisional of application: 00945200.4
(73) Proprietor: Smith Medical ASD, Inc., Rockland, MA 02370 (US)
(72) Inventor: Brunner, Charles, North Reading, MA 01864 (US); Plaisted, Richard, Cataumet, MA 02534 (US); MacDougall, Peter, Cambridge, MA 02141 (US); Waldron, Vincent, Hull, MA 02045 (US); Zarkin, Eugene B., Boston, MA 02144 (US); Kyn, Pavel, Somerville, MA 02134 (US)
(74) Representative: Hedges, Martin Nicholas

(56) References cited:
- EP-A- 0 463 837
- US-A- 3 383 085
- US-A- 4 678 460
- US-A- 4 759 749
- US-A- 4 874 359

## Description

The present invention relates to a connector assembly integrated into a temperature regulating device for interconnecting a fluid conduit to the device so that a fluid of a desired temperature can be provided via the fluid conduit to a patient.

Devices for warming physiological fluids, such as for example whole blood and packed cells, to a desired temperature before providing the warmed physiological fluids to a patient are well known. Once such prior art device is the HOTLINE® system made by the assignee of the instant invention. Such a device uses a heater and hardwired control for heating a fluid such as for example water to a desire temperature, so that the water can in turn warm the infusate to be provided to the patient via a fluid conduit. Inasmuch as the feedback for such system is hardwired, its feedback response is somewhat limited. Moreover, given the limitation of the display mechanism in the prior art HOTLINE® device, no meaningful interfacing between the device and the user is achieved.

EP-A-0463837, upon which the precharacterising clause of claim 1 is based, discloses a connector assembly for coupling a heat exchange conduit to a fluid temperature regulation unit, comprising: mounting means mounted at a given location to said fluid temperature regulation unit to provide a communications path for a fluid whose temperature is regulated by said unit to traverse out of and into said unit; and connector means having coupled thereto said heat exchange conduit matable with said mounting means, said connector means having one port means for receiving and routing said fluid from said fluid temperature regulation unit to said heat exchange conduit and another port means for returning said fluid from said heat exchange conduit to said fluid temperature regulation unit, characterised in that said mounting means is mounted coplanarly along one wall of said unit, said one and other port means being configured in said connector means to complete said communications path provided by said mounting means when said connector means is demountably engaged to and sealingly mated with said mounting means by said connector means being moved to a predetermined location coplanarly along said one wall relative to said mounting means.

According to the present invention there is provided a connector assembly for coupling a heat exchange conduit to a fluid temperature regulation unit, comprising: mounting means mounted at a given location to said fluid temperature regulation unit to provide a communications path for a fluid whose temperature is regulated by said unit to traverse out of and into said unit; and connector means having coupled thereto said heat exchange conduit matable with said mounting means, said connector means having one port means for receiving and routing said fluid from said fluid temperature regulation unit to said heat exchange conduit and another port means for returning said fluid from said heat exchange conduit to said fluid temperature regulation unit, characterised in that said mounting means is mounted coplanarly along one wall of said unit, said one and other port means being configured in said connector means to complete said communications path provided by said mounting means when said connector means is demountably engaged to and sealingly mated with said mounting means by said connector means being moved to a predetermined location coplanarly along said one wall relative to said mounting means.

A connector assembly in accordance with the invention has the advantage that it is designed in such a way that the circulation of fluid through the fluid conduit occurs only if the fluid conduit is correctly mated to the connector assembly. To test the integrity of the temperature regulating section, the fluid conduit can be replaced by a temperature calibration or checking device, Once the temperature checking device correctly fits to the connector assembly, a test takes place for determining the calibration and integrity of the various temperature sensing components in a temperature regulating section, to therefore ensure that the device is operating properly.

In order that the invention may be well understood, there will now be described an embodiment thereof, given by way of example, reference being made to the accompanying drawings, in which:
Fig. 1 is a block diagram illustrating the various components of a device of which the connector assembly of the present invention forms a part;
Fig. 2 is a flow diagram providing an overall view of the various functions of the device of Figure 1 ;
Fig. 3 is a flow diagram illustrating the Power On and Self Test procedures of the device of Figure 1 ;
Fig. 4 is a flow chart illustrating the Normal Operation and Initialization processes of the device of Figure 1;
Fig. 5 is a flow chart that illustrates the Main Control Loop process of the device of Figure 1
Fig. 6 is a flow chart illustrating the Turning Off process of the device;
Fig. 7 is a perspective view of a connector assembly embodying the instant invention, as it is incorporated into the housing of the device of Figure 1 ;
Fig. 8 is a perspective exposed view of the various components of the connector assembly of the instant invention;
Fig. 9 is an enlarged view of the components that make up the connector assembly of the instant invention;
Fig. 10a is a perspective view of the support plate of the connector assembly of the instant invention which is mounted to the back of an opening of the housing to which bi-directional receptacle ports are located;
Fig. 10b is a perspective bottom view of the support plate of the connector assembly of Fig. 10a;
Fig. 11 a to 11 c are respective perspective views of the housing plate of the connector assembly of the instant invention;
Fig. 12 is a perspective semi-exposed view of the connector body of the connector assembly of the instant invention to which is connected a fluid conduit through which the infusate to be provided to the patient flows;
Fig. 13 is another perspective view of the connector body of the Fig. 12;
Fig.14 is a perspective bottom view of the connector body of Fig. 12 showing the routes through which the fluid is circulated in the connector body;
Fig. 15a is a plan view of the housing of the device with the connector assembly being integrated thereto;
Fig. 15b is an enlarged view of the connector assembly and the mating thereto of the connector of Fig. 12;
Fig. 16 is a flow diagram illustrating the self-testing procedure of the device and
Fig. 17 is a flow chart illustrating the operation of the Bio-Test that takes place when a calibration connector device is mated to the connector assembly of the instant invention.

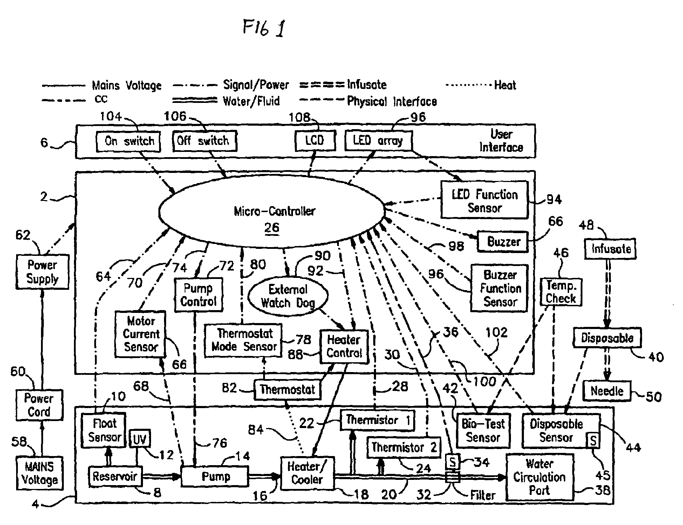

With reference to Fig. 1, a temperature regulating device with which the connector assembly of the present invention is used is shown to include three major sections a controller section 2, a temperature regulation section 4 and a user interface section 6. The three sections are communicatively interconnected.

As shown, temperature regulation section 4 has a reservoir 8 for storing the fluid that is to be temperature regulated. The fluid could be any type of fluid including non-compressible fluid or gas. But for the discussion of the exemplar embodiment, water is the preferred fluid to be used. Reservoir 8 has communicatively connected thereto a float sensor 10 that senses the level of fluid in reservoir 8. There may also be positioned relative to reservoir 8 an ultraviolet light source 12 which could direct an ultraviolet light to the fluid in the reservoir for killing any bacteria and/or microbes that may find their way into the fluid stored in reservoir 8. Note that ultraviolet sensor 12 may also be positioned along the tubings that connect to the bidirectional inlet/outlet ports in the temperature regulation section 4 for killing any bacteria and/or microbes that may be in the tubings and/or in the fluid that flows through the tubings.

Reservoir 8 is connected to a pump 14, which is a bidirectional pump or equivalents thereof that may be purchased from a number of companies including the Gorman-Rupp Company. In essence, pump 14 could be a bidirectional positive displacement gear pump that can circulate the fluid in either a forward direction, as indicated for example by arrow 16, or in a reverse direction so as to route the fluid back into reservoir 8.

Pump 14 is connected to a heater, or cooler, 18 which, in the instance that it is operating as a heater, warms the fluid circulated thereto by pump 14 to a desired temperature. Alternatively, the fluid output from pump 14 may also be cooled by cooler 18 to a desired temperature. For ease of understanding, assume for the rest of this discussion, heater, or cooler, 18 works as a heater for warming a fluid output from pump 14.

The fluid, in this instance water, output from heater 18 is routed to a tubing 20 that has communicatively connected thereto two thermistors 22 and 24. These thermistors each independently sense the temperature of the fluid being heated by heater 18 and flowing along tubing 20. Respective signals are output by the thermistors to processor 26 via signal or channel lines 28 and 30. Thermistors 22 and 24 are each calibrated to a set point, so that theoretically each of the thermistors should measure the same temperature from the fluid flowing along tubing 20. If for any reason the respective temperatures measured by thermistors 22 and 24 diverge for more than a predetermined amount, such as for example 3°C, then processor 26, upon sensing this variance, would shut the system down. This is done in order to ensure that the temperature of the fluid being heated by heater 18 remains accurate.

Thermistors 22 and 24 each also work cooperatively with processor 26 to ensure that the system does not reach an overheated or overtemp condition. This is done by thermistors 22 and 24 each continuously reporting to processor 26 whether a temperature higher than the normal operating temperature, i.e., an "overtemp" condition such as for example 2°C above the normal temperature, has been sensed. If either one of thermistors 22 and 24 senses such overtemp condition, the heater is shut down.

Alternatively, each of thermistors 22 and 24 is set to have its circuit electrically respond to the overtemp condition so as to automatically shut down the operation of heater 18. Once the system has been shut down due to an overtemp condition, the machine would remain in such fail-safe shut down mode.

To maintain the desired temperature of the fluid, one of thermistors 22 and 24 is used to activate a control relay to turn heater 18 "on" and "off" in response to the temperature of the fluid to thereby maintain the temperature of the heated fluid to be within an acceptable temperature range.

Further along tubing 20 there may be positioned a filter 32 that could filter out any bacteria and/or microbes that might be in the fluid. Filter 32 may have connected thereto a sensor 34, which may continuously monitor the filtering element of filter 32 for ensuring that the filter element continues to be able to filter out microbes and bacteria. Sensor 32 may do this by detecting the opacity of the filter element in filter 32. A signal from sensor 34 is fed to processor 26 by means of a signal line 36.

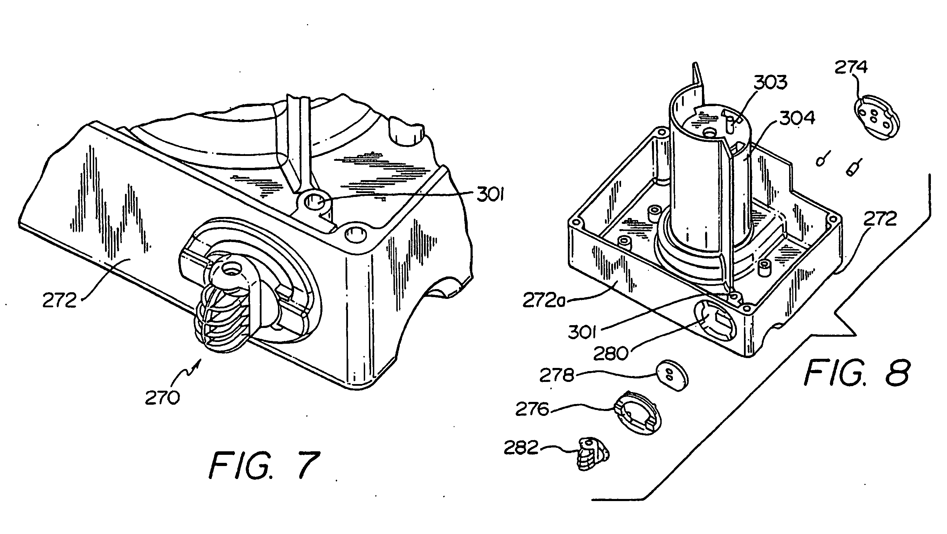

The fluid from tubing 20 is routed to a water circulation port 38 that comprises inlet/outlet bidirectional ports that enable the fluid from tubing 20 to be routed to a fluid conduit in the form of a disposable 40. Disposable 40 may be a triple lumen conduit such as that described in U.S.-A-5,097,898 and US-A-5,063,994. Although not shown in Fig. 1, the mating of disposable 40 to bidirectional ports 38 is done by means of a connector assembly such as for example that shown in Figs. 7-15. A more detailed discussion of the connector assembly and the connector mated thereto from which disposable 40 extends will be given, infra.

Return to Fig. 1. Note that there are a Bio-Test sensor 42 and a disposable sensor 44 each positioned relative to the bidirectional ports and the connector assembly as mentioned above. Sensor 42 and 44 will be discussed in detail with the discussion of the connector assembly. For now it suffices to say that Bio-Test sensor 42 is an electromagnetic sensor that senses the mating to the connector assembly of a calibration device such as a Temp Check 46 for initiating a test that determines the integrity of various components in temperature regulating section 4. Disposable sensor 44, on the other hand, is an electromechanical switch or sensor that continuously monitors whether the fluid conduit, i.e., disposable 40, or Temp Check 46 is fitted correctly to the connector assembly. If either of disposable 40 or Temp Check 46 is not mated to the connector assembly properly, sensor 42 sends a signal to processor 26, which in turn prevents the instant invention device from working.

Moreover, an additional sensor 45, which may be either an electromagnetic sensor or an electromechanical switch positioned relative to the connector assembly, continuously monitors the integrity of the connector assembly. In the event that tampering of the connector assembly, such as for example the removal thereof, is detected, sensor 45 would send a signal to processor 26 to inform processor 26 to disable the device. This could be done by processor 26 sending a signal such as for example a delete signal to an EPROM, wherein various functional algorithms relating to the operation of the device are stored, to erase at least one of the algorithms that operates the device. Thereafter, to reenable the device, the device has to be returned to the factory so that the erased algorithm could be reinstored.

With respect to disposable 40, note that it includes the conduit to which infusate 48 is input so that the infusate can be provided to a needle 50, which is used to infuse the patient. Infusate 48, as it flows through disposable 40, is warmed to the desired temperature by the fluid that is being circulated from pump 14 to disposable 40.

Controller section 2 of the instant invention device has connected to its processor 26 a number of components. For example, a signal is provided to processor 26 from float sensor 10 via line 64. As mentioned previously, float sensor 10 senses the level of fluid in reservoir 8, so that if the level happens to fall below a predetermined level, processor 26 can notify the user that additional fluid is required via some indicator such as for example a buzzer 66 and/or a light at LED array 96. Moreover, if float sensor 18 were to sense the level of fluid in reservoir to be below a minimum level, it will send a signal to processor 26, which will then shut the system down.

Processor 26 has also input thereto a signal from a motor current sensor 66 that senses the current of pump 14 by way of a signal line 68 to pump 14. The value of the sensed current informs processor 26 of the operational status of pump 14. To wit, if sensor 66 senses a normal current, for example from 100-500 µA, then processor 26 knows that pump 14 is operating normally. However, if the current is sensed to be above 500 µA, for example between 500 and 800 µA, then processor 26 knows that something is wrong. Accordingly, a "NOT WARMING" warning is displayed to the user. At this stage, the problem could be one that is readily fixable, such as for example disposable 40 having a kink that is easily straightened out. Finally, if the sensed current from pump 14 has a value greater than a predetermined amount, for example 800 µA, then processor 26 knows that something is wrong with the motor of pump 14. At this time, in addition to displaying the "NOT WARMING" warning, processor 26 could also shut down pump 14 to prevent any potential damage thereto.

Processor 26 is further connected to a pump control 72 by means of a signal line 74. Pump control 72 controls the direction of rotation of pump 14 for circulation of the fluid in either the forward direction, as exemplified by arrow 16, or the reverse direction so as to drain the fluid in disposable 40 and tubing 20 back into reservoir 8. In receipt of a turn off signal, processor 26 would instruct pump control 72 to reverse the rotation of pump 14 to thereby drain the fluid in tubing 20 and disposable 40 back into reservoir 8.

A thermostat mode sensor 78 is communicatively connected to processor 26 via a signal line 80. Thermostat mode sensor 78 is also connected to an external thermostat 82, which continuously monitors the operating temperature of heater 18 via a signal line 84. Thermostat 82 is a "fail-sale" device in that it is preset to a given temperature that, if sensed, causes it to shut down the system so as to prevent any catastrophic damage to the device and potential harm to the patient. Thermostat 82 therefore acts as a backup to thermistors 22 and 24 in the event that both of those thermistors fail. Thus, the shut down temperature in thermostat 82 is preset to a value that is higher than the set point temperature and the overtemp condition thermistors 22 and 24 each were calibrated to respond to, and quite a bit higher than what is considered to be the operating temperature of heater 18.

In the meantime, whether thermostat 82 is operating properly is periodically being monitored by thermostat mode sensor 78. Sensor 78 could be an inductor coil placed about the conductor that provides power to thermostat 82 so as to sense the current flowing through thermostat 82. If per chance thermostat 82 fails, sensor 78 can sense that there is no current flowing therethrough. It will accordingly inform processor 26 that thermostat 82 has malfunctioned.

An external watchdog module 90, which is an electrical circuit that comprises a Schmidt trigger and its own time base, is communicatively connected to processor 26. Watchdog 90 continuously monitors the operation of processor 26 per its timing and compares the time base of processor 26 with its own clock pulses to ensure that processor 26 is operating properly. Watchdog 90 would shut the system down if it senses that processor 26 is not functioning properly by cutting off power to heater 18, such as for example by opening a safety relay that connects heater 18 to its power source. Note that in addition to external watchdog 90, there is an internal watchdog in controller 26 that monitors the execution of the software of the system.

Other components connected to processor 26 that reside on section 2 include an LED function sensor 94 that senses the operation of LED array 96 located in user interface section 6. Buzzer 66, as mentioned previously, may be used to alert the user of problems in the device of the instant invention that he or she should be made aware of. In particular, buzzer 66 outputs different sound signals for different problems, as for example three beeps or a specific tone for an "OVERTEMP" condition as compared to one beep or another tone for a "NOT WARMING" condition. A buzzer function sensor 96 senses the signals output from buzzer 66 and provides a feedback to processor 26 by means of a feedback signal line 98.

Processor 26 is further communicatively connected to Bio-Test sensor 42 and disposable sensor 44 by means of signal lines 100 and 102, respectively. When a signal is sent by Bio-Test sensor 42 to processor 26 to inform it that Temp Check 46 is in place, processor 26 begins a calibration and temperature testing procedure for determining the integrity of various components of the system. These components include thermistors 22, 24 and thermostat 82.

In particular, each of thermistors 22 and 24 is calibrated to respond to a given set point, for example 41.9°C, that the temperature of the fluid is to be regulated at. Moreover, thermistors 22 and 24 each are tested to ensure that they each will electrically respond to an "overtemp" condition. An overtemp condition may for example be set to occur if the temperature of the fluid is measured to be 1.5°C above the set point. When either of thermistors 22 and 24 senses the "overtemp" condition, heater 18 is shut down.

Thermostat 82 is tested separably from both thermistors 22 and 24. Bypassing thermistors 22 and 24, thermostat 82 is tested to determine if it will open at a predetermined fail-safe temperature that is well above the temperature at which thermistors 22 and 24 each become electrically open. In other words, by making sure that thermostat 82 will open at the failsafe temperature, the system is ensured to shut down at that predetermined fail-safe temperature to prevent any catastrophic occurrences.

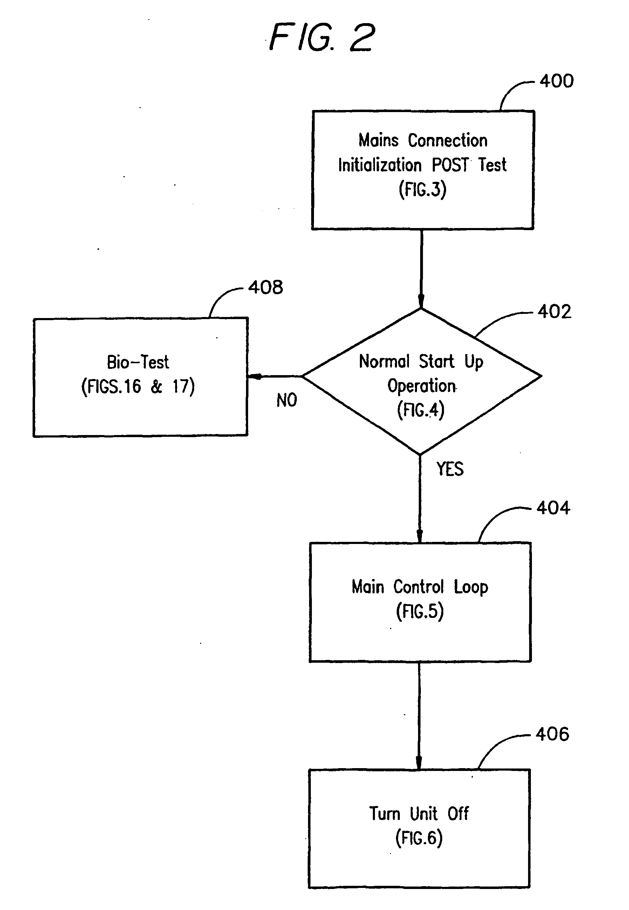

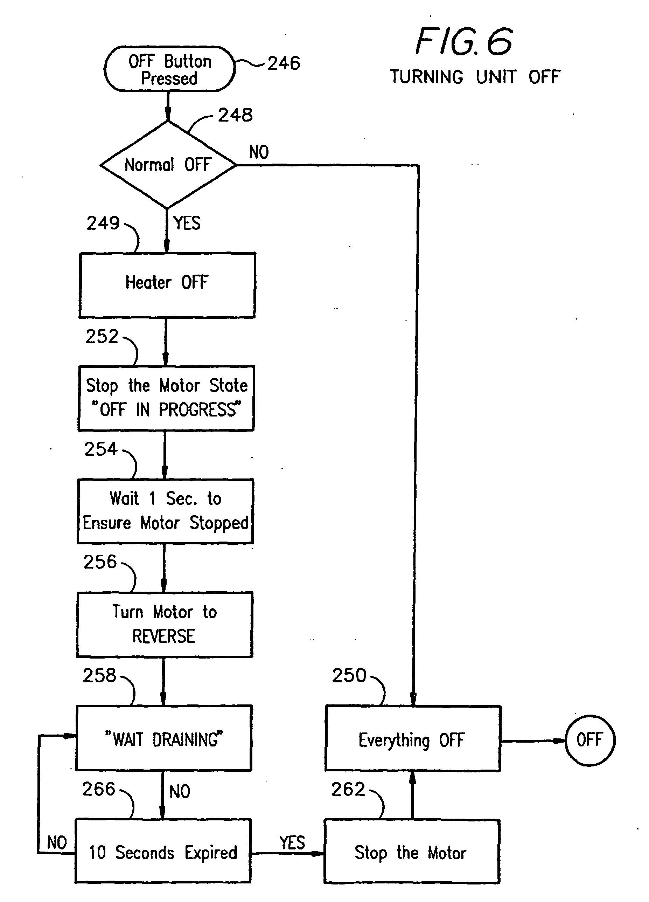

The last major section in the device is user interface section 6. As shown, user interface section 6 has an on switch 104, an off switch 106, a LCD display 108, as well as the previously mentioned LED array 96. The on and off switches 104, 106 are switches that, once activated, would signal processor 26 to take different predetermined courses of action, which will be discussed in detail with respect to the flow diagrams of Figs. 2 and 6. LCD display 108 is a display that provides to the user messages in any one of a plurality of languages so that the device of the instant invention can be shipped to various countries without having to have its menus and instructions reprogrammed specifically for the country that it is to be shipped to. The interaction between LCD display 108 and controller 28 is conventional in that the typical drivers and signal generators, as well as conventional memory stores such as ROMs that store the different languages and menus, are used. LED array 96, as was discussed previously, provides the user an indication of the types of functions that the device is performing. A touch sensitive screen or input keys provide the interfacing between the user and the instant invention device.

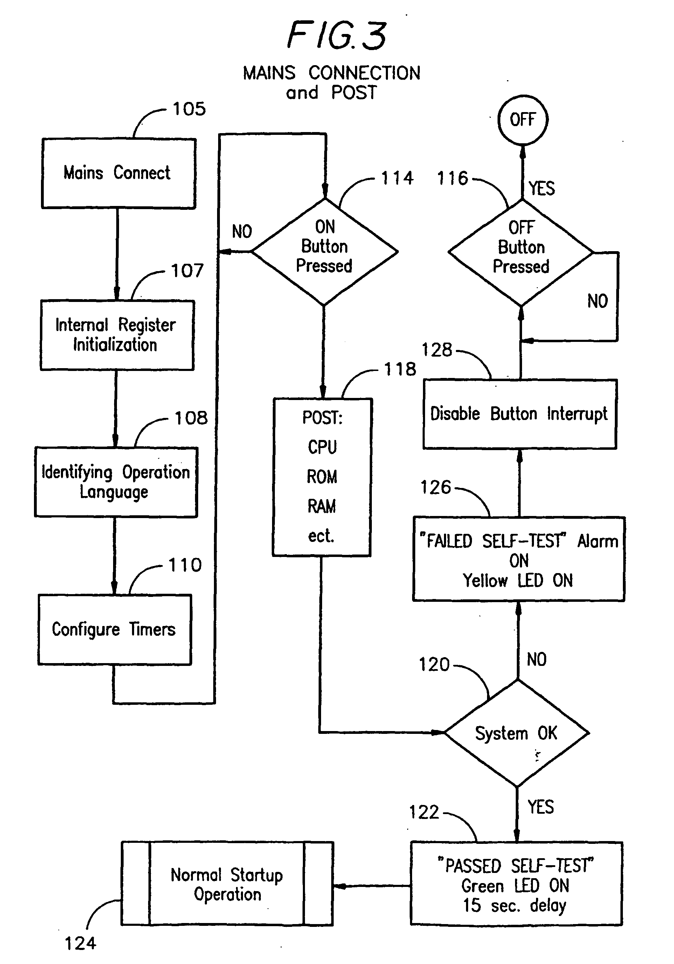

An overall top level flow diagram illustrating the main operation processes of the system is shown in Fig. 2 As shown, there are five major processes. These are: Mains Connection, Initialization and Power On Testing process 400; Normal Operation Process 402; Main Control process 404; Turn Off process 406 and Bio-test process 408. These processes are to be discussed hereinbelow with reference to Figs. 3-6.

With reference to Fig. 3, a combination Mains Connection and Power On Self Test (POST) flow chart is provided. The Power On process begins when the mains are connected at step 105. The POST process is initiated at step 114 when ON switch 104 is pressed. Briefly, the power on test is a self test to be performed by the system to test its various components such as for example its RAM, ROM and any other component that may deal with the safety or temperature regulating aspect of the device. As its name implies, every time that the machine is turned on, the testing of the various components of the system is performed.

In particular, starting at step105, mains are connected. Thereafter, an initialization test 107 is performed in the read only memory (ROM) of the device in which the various programs or software routines for performing the various functions of the device is tested. A test is also performed on the random access memory (RAM) of the device. The internal register of the device is also initialized. Such internal register initialization also includes the initialization of the LCD display and the EEPROM. Note that such ROM, RAM, EEPROM and registers are common in electronic devices and therefore are not shown in the block diagram of the device as illustrated in Fig. 1.

Following the initialization in step 107, the process proceeds to step 108 in which the operation language is identified. This operation language could be any one of a plurality of languages stored in a memory in the device so that the device can be sold in most of the countries of the world.

In step 110, the timers are initialized. Internal watchdog operation begins to monitor program flow. Note that steps 107-110 do not need to be processed in the particular order shown.

Next, the process waits until on button 104 is pushed, per step 114. When the on button is pushed, the system proceeds to step 118 to perform the POST test for determining the integrity of the various components of the system. This POST process includes testing of all components associated with the safety of the system such as the CPU, RAM, ROM, etc. If the system tests out okay per step 120, the appropriate green LED at LED array 96 is lit, per step 122, to indicate to the user that the system is operational. Thereafter, per step 124, the device proceeds to perform the normal operation, and the POST process is completed.

However, if the system tests abnormal in step 120, a failed self test alarm, as indicated by a yellow LED in LED array 96, is lit to indicate to the user that the self test has failed. If there is a failed self test, the process proceeds to step 128 to output a disabled interrupt to the processor for disabling the system. The process then waits until the off button is pressed by the user per step 116 to turn off the system.

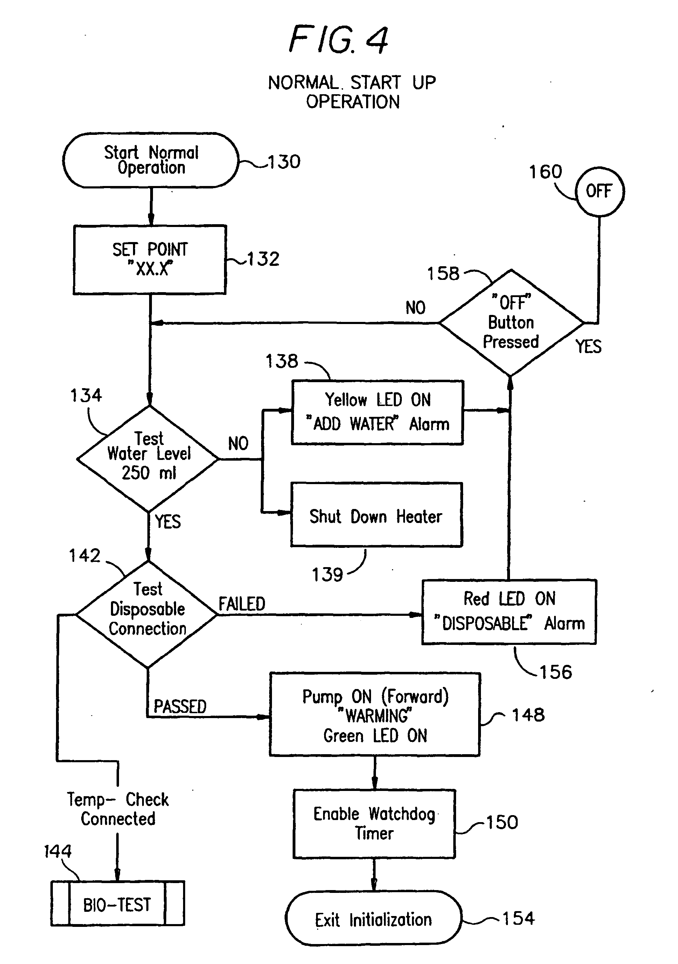

The Normal Start Up Operation process is illustrated in Fig. 4, A predetermined set point temperature is provided by the user and read at step 132. At step 134, the fluid level is tested to determine whether it is at least 250 ml, the minimum fluid level allowing the machine to operate. Any level lower, the machine will shut down. If the fluid level is sensed to be between 250 ml and 300 ml, fluid needs to be added to reservoir 8. Thus, if per step 134, the water level is determined to be less than 250ml, a yellow LED is lit on LED array 96 to indicate to the user that he or she should add fluid, per step 138. At the same time, power is shut off for heater 18 per step 139. The device, in the meantime, will not operate. On the other hand, if the level of fluid is above 250 ml but below 300 ml, the yellow LED is lit on LED array 96 to indicate to the user that the fluid level in reservoir 8 is low, and the message "Water Tank Low" is flashed periodically on the LCD display.

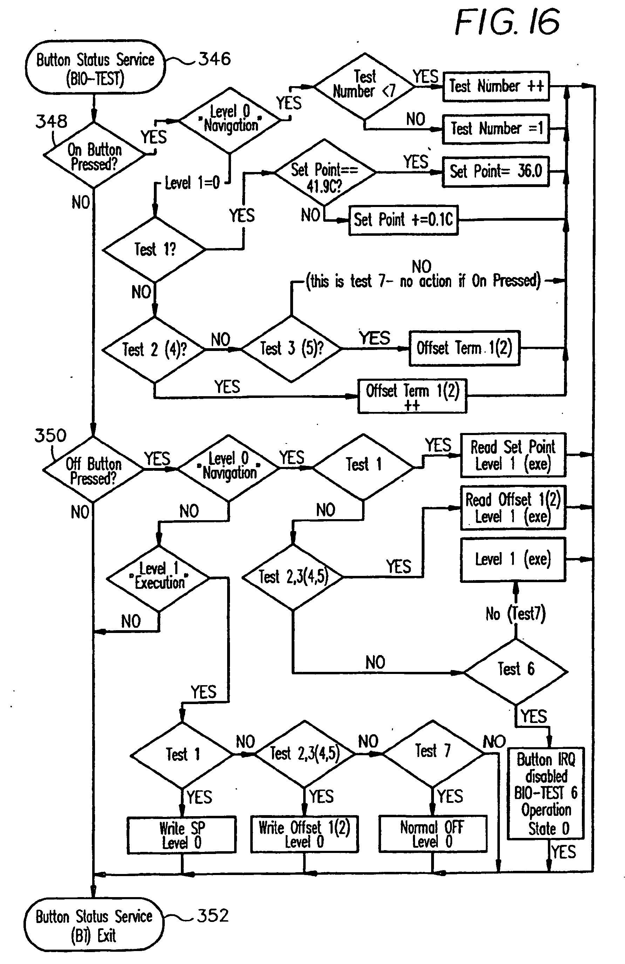

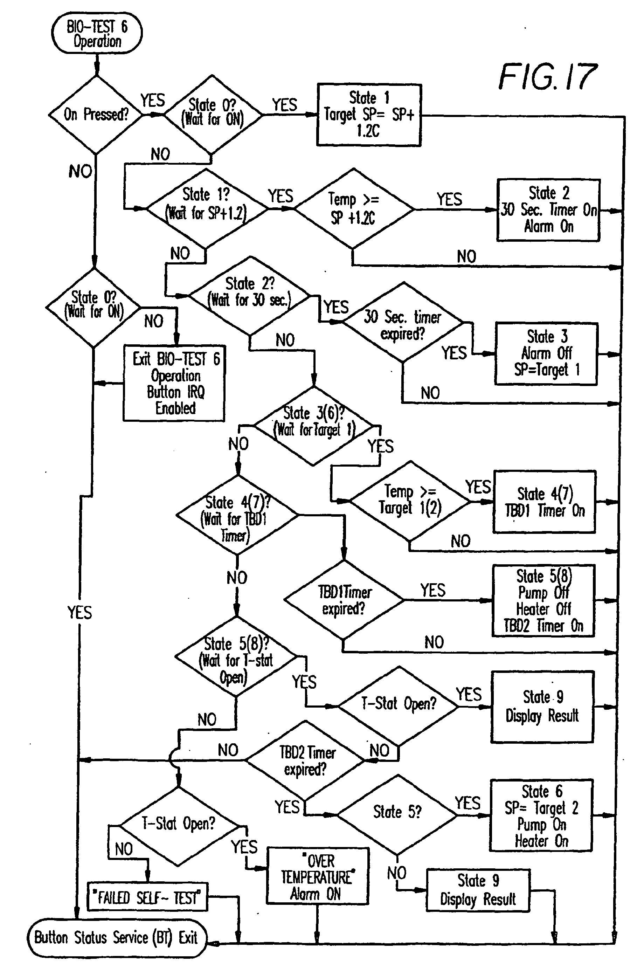

The next step of the normal start up operation process is step 142, which tests whether the disposable fluid conduit has been properly connected to the connector assembly. If it turns out that Temp Check 46 is properly fitted to the connector assembly, the "Bio-Test" of the system, which is shown in much greater detail in Figs. 16 and 17, begins, per step 144.

On the other hand, if a fluid conduit is determined to have been properly connected to the connector assembly per step 142, the process proceeds to step 148 to initialize pump 14 to circulate the temperature regulated fluid, and light the green LED on LED array 96 to indicate to the user that the device is operating normally. Thereafter, the timer for external watchdog 90 is enabled, per step 150. After which the operation process exits from its start up phase, per step 154.

Return to step 142 where a determination is made on whether the connector for the fluid conduit has been properly connected to the connector assembly. If it is determined that the fluid conduit connector is not properly connected, a yellow LED relating to disposable fluid conduit 40 is lit on LED array 96 to indicate to the user that disposable 40 is not properly connected, per step 156. Thereafter, the process waits for the activation of the off switch, per step 158. Once the off button is pressed per step 158, the device is turned off, per step 160.

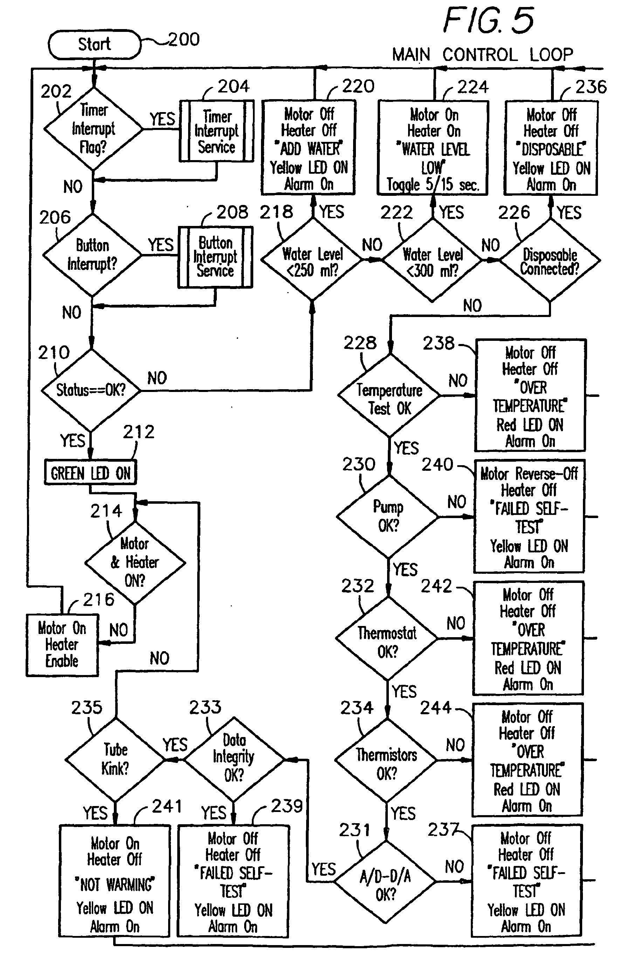

After the normal start up process as illustrated in the flow chart of Fig. 4, the operation of the device proceeds to the Main Control Loop process as illustrated in the flow chart of Fig. 5. From the start step 200, the process proceeds to step 202 for determining whether the timer interrupt flag has been raised. If it has, the process proceeds to timer interrupt service per step 204 for clearing the timer interrupt. If there is no timer interrupt, the process proceeds to step 206 to determine whether any buttons have been pushed. If a button has been pushed, the process proceeds to step 208 for the user to do what he or she needs to do to clear the button interrupt. The process then proceeds to step 210 at which time the system determines whether its overall status is okay. If it is, the green LED at LED array 96 is lit, per step 212 to signify that the system is operational.

Thereafter, a determination is made on whether pump 14 and heater 18 each have been turned on, per step 214. If yes, the process proceeds back to the beginning of the loop process to wait for an interrupt from either the timer or the off switch, per steps 202 and 204, respectively. If on the other hand, per step 214, it is determined that pump 14 and heater 18 have not been turned on, the process proceeds to step 216 to turn on pump 14 and heater 18. The process then proceeds to the beginning of the loop to wait for either the timer interrupt or the button interrupt, as indicated per steps 202 or 206, respectively.

As mentioned previously, provided that the status of the device remains okay at step 210, the device is deemed to be operational and the green LED is lit per step 212. However, if it is determined per step 210 that any one of the tests as shown in Fig. 5 has failed, then a number of additional tests will take place. This is illustrated starting with step 218 in which a determination is made on whether the fluid level is less than the minimum acceptable level. If it is less than the minimal acceptable level, pump 14 and heater 18 are each turned off, per step 220. Also, per step 220, the "add water" yellow LED on LED array 96 is lit to warn the operator that more water or fluid is required. The process thereafter returns to the beginning of the loop to once again wait for interrupts or any sign that the status of the device is acceptable.

Other tests are continuously being performed on the device. For example, after the minimum fluid level test at step 218 is performed, if it is determined that reservoir 8 does have the minimum fluid level at step 18, the process proceeds to step 222 to determine whether the fluid level at reservoir 8 has reached the maximum fluid level. If it has not, a yellow LED is lit at LED array 96 to indicate to the user that the water level is low, per step 224. In this instance, the device remains operational, as the process proceeds to step 214 to determine the operational status of pump 14 and heater 18.

Other tests after the status of the device has been deemed not to be okay include: testing the connection of the fluid exchange conduit with the connector assembly per step 226, testing the temperature per step 228, testing the operation of pump 14 per step 230, testing thermostat 82 per step 232, testing thermistors 22 and 24 per step 234, testing the A/D and D/A converters per step 231, testing of data integrity per step 233 and testing for kinks in the conduit per step 235. If each of those tests shows that the status of the device is acceptable, the process proceeds to step 214 for determining whether pump 14 and heater 18 are each turned on. However, if any one of tests 226-235 fails, pump 14 and heater 18 each are turned off, as indicated per each of steps 236-244. Also, the appropriate LED at LED array 96 is lit for whichever test has failed at each of steps 236-244. The process of Fig. 5 continues until either the time for the device to remain operational has run out or until the off button is pushed, per steps 202 and 206, respectively.

With respect to tests 218 to 235, note in particular the following. Test 234 of the thermistors proceeds with the reading of the first thermistor 22. The temperature of the second thermistor 24 is then read. The respective temperatures of the thermistors are then compared. If both temperatures are the same, the power to heater 18 is turned on. On the other hand, if the respective temperatures of the thermistors are not the same, the system status fail flag for the thermistors is raised to activate a yellow LED to warn the user that a problem exists per step 244. At the same time, a "Fail Self Test" message is displayed to the user.

Another test that may require some clarification is the A/D and D/A test 231. This test involves the periodic cross checking of the analog to digital converter with the digital to analog converter to insure the integrity of the respective converters for signal conversion.

Yet another test that is worth discussing is the data integrity test 233. There all critical data including the exemplar set point 41.9°C is read by using two independent routines. A comparison test is then performed on the data. If the comparison test fails, then the yellow LED is turned on, the message "Failed Self-Test" displayed and the appropriate alarm sequence is generated by buzzer 66. Moreover, pump 14 and heater 18 are each turned off.

Fig. 6 illustrates the process when the off button is pressed. Starting with step 246 which indicates that the off button is pressed, the process proceeds to a determination, per step 248, on whether the turning off is a "normal" off. If it is not, every component in the system is turned off per step 250, as the system is programed to assume that something catastrophic may have happened. Conversely, if the system determines that it is a "normal" off, the process proceeds to step 249 to turn off heater 18, and then step 252 to turn off the motor for pump 14. Next, per step 254, the system waits a predetermined time period, for example 1 second, to make sure that the motor for pump 14 has stopped.

Thereafter, per step 256, pump 14 is turned on in reverse, so as to reverse the circulation movement of the fluid in the system including the fluid conduit connected to the connector assembly. This is signified per step 258 whereby the fluid in both tubing 20 and the disposable fluid conduit 40 is pumped back to reservoir 8. The reverse circulation process continues for a given time period, such as for example 10 seconds, as indicated per step 266. At which time disposable fluid conduit 40 may be removed from the connector assembly, as pump 14 stops operation per step 262. A "Remove Disposable" message may also be displayed to the user. A time delay such as for example 15 second may be programmed into the system to ensure that the various components are turned off in an orderly fashion. After that, the device shuts down per step 250.

The connector assembly of the instant invention, as it relates to the device previous by described is best shown in the perspective views of Figs. 7, 8 and 9. As illustrated, connector assembly 270 is mounted to housing 272 of the device

There are a number of components that make up the connector assembly. These include a back plate 274 which is mounted to the back of wall 272a of housing 272. Back plate 274 is secured to a housing plate 276 positioned to the exterior of wall 272a. Thus, back plate 274 and housing plate 276 are secured to each other at opposite sides of opening 280 at wall 272a as housing plate 276 superposes over back plate 274. A sealing gasket 278 is also part of the connector assembly, as it sealingly fits within an opening 282 at back plate 274 encircled by a circumferential wall 284 extending away from a flange 292 of back plate 274.

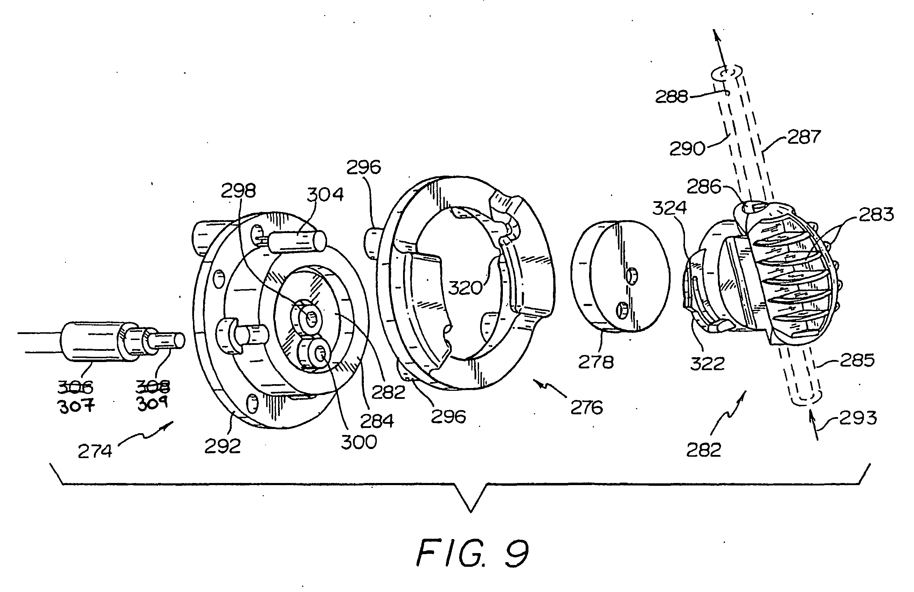

The connector for the disposable fluid conduit is shown in Figs. 7-9 as component 282, or otherwise referred to in the instant invention as a reflux connector. As best shown in Fig. 9 per the dotted lines, a disposal or fluid conduit 284 is connected to an opening 286 of reflux connector 282. Fluid conduit 287, as is disclosed in the above incorporated by reference '898 patent, is a plastic tubing that has an inner flexible plastic tube 288 surrounded concentrically by an outer flexible plastic tube 290. The infusate to be provided to the patient is input to tube 288 per arrow 293 via another conduit or tubing 285 at another opening of reflux connector 282. The infusate, as it is being supplied to the patient, is warmed by the temperature regulated fluid that flows through tube 290.

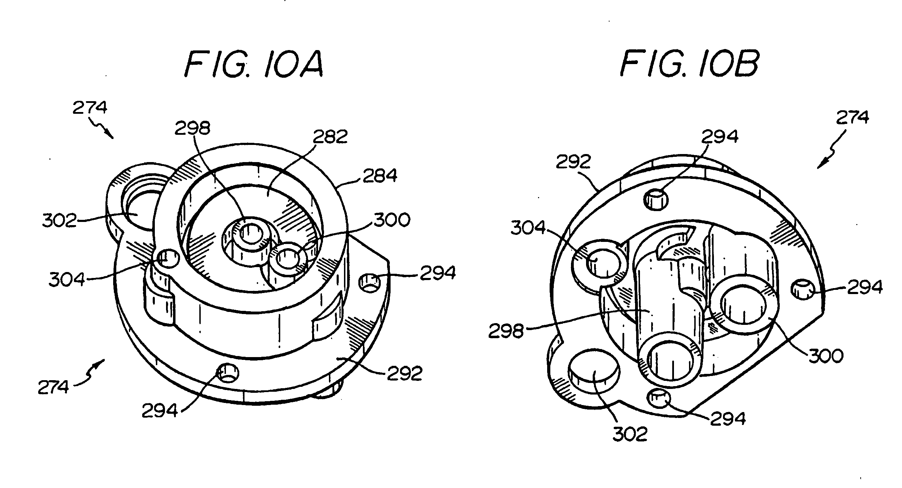

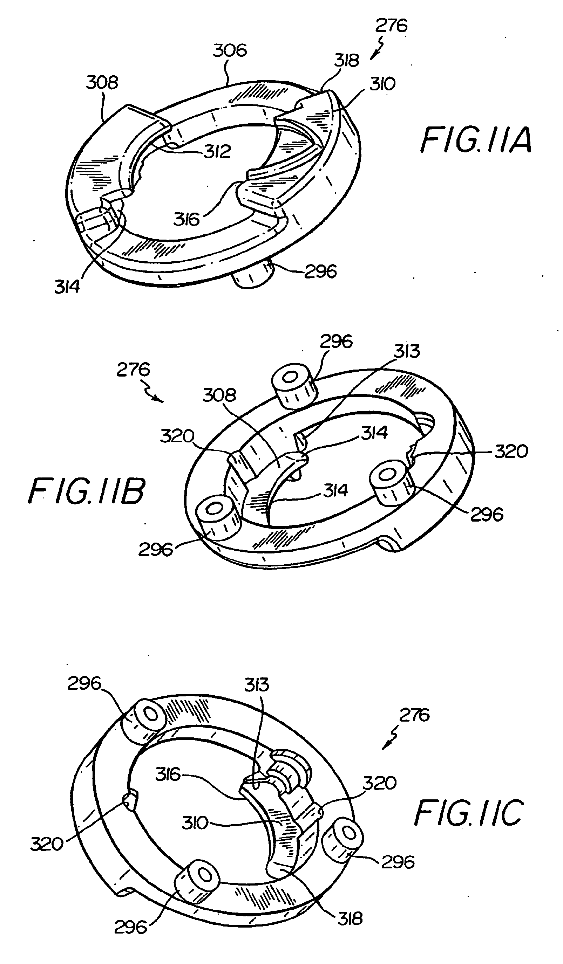

As best shown in Figs. 10a and 10b, back plate 274 of the connector assembly of the instant invention is a one-piece molded plastic member mounted to the inside of wall 272a of housing 272 via flange 292. This is done by aligning a plurality of holes 294, provided along flange 292 of back plate 274, with a corresponding number of internally threaded supports 296 extending from the back of housing plate 276. See Figs.11 a-11 c. By aligning the proper support 296 with the appropriate hole 294, housing plate 276 superposes over back plate 294 at hole 280 of housing 272. A plurality of screws or other fastening means, not shown, tighten housing plate 276 to back plate 274, while at the same time anchor both back plate 274 and housing plate 276 to wall 272a of housing 272 of the device of the instant invention.

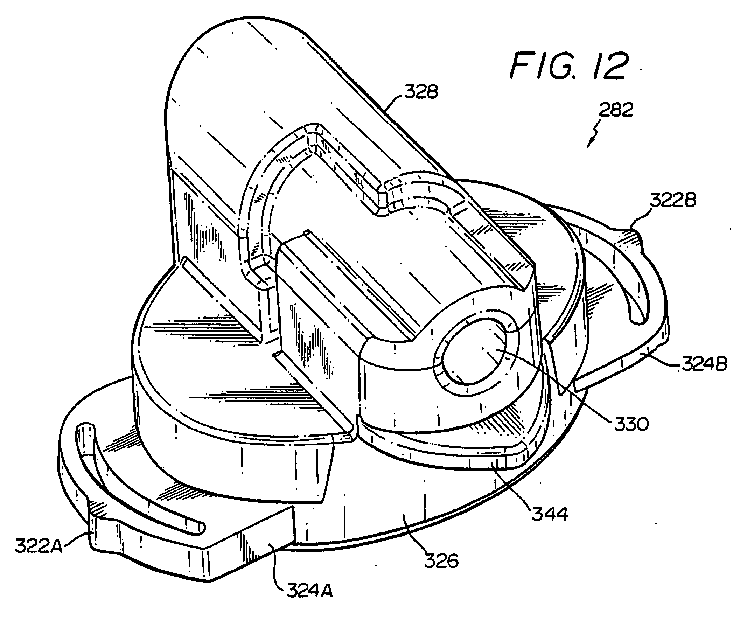

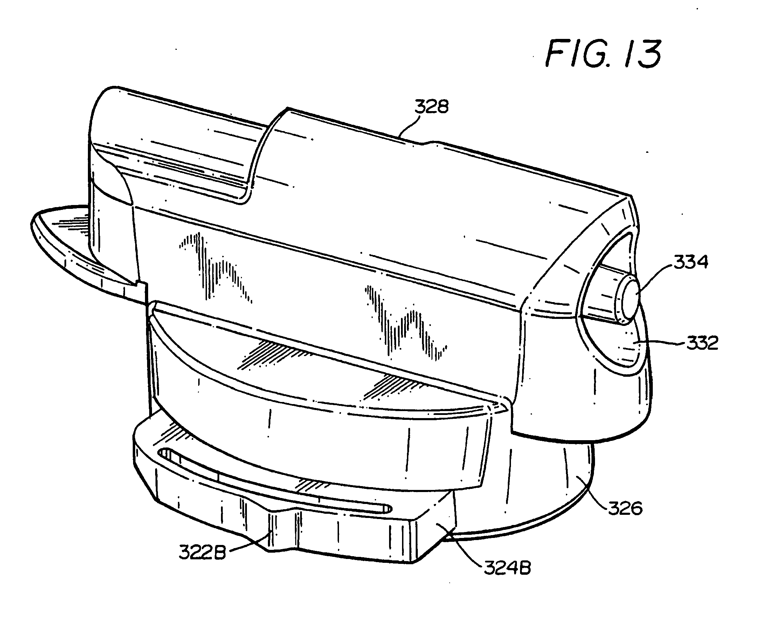

Note that the various components of the connector assembly shown in Figs. 7-9 may be cosmetically somewhat different from those shown in Figs. 10, 11, 12, 13 and 14. This is because the components shown in Figs.7-9, even though they are functionally the same as those shown in the later figures, in fact represent a different embodiment of the connector assembly. For example, connector 282 as shown in Figs. 7-9 has at its top portion a number of fins 283 which are not germane to the inventiveness of the instant invention and accordingly are not shown in the connector 282 as illustrated in Figs. 12 and 13.

With more particular focus to backplate 274 as shown in Figs. 9, 10a and 10b, note that within opening 282 surrounded by circumferential wall 284 there are provided inlet/outlet ports 298 and 300. Ports 298 and 300 are the supply and return (or reflux) conduits that could be considered as part of the water circulation port 38 shown in Fig. 1. Thus, by way of supply port 298, the temperature regulated fluid, be it water or otherwise, is fed to fluid conduit 287, and specifically the outer tube 290 that surrounds inner tube 288, for warming the infusate that flows through inner tube 288. Return port 300 provides a path whereby the fluid being used to temperature regulate the infusate is circulated back to heater/cooler 18 for more heating and/or cooling, so that the fluid that is used for maintaining a desired temperature for the infusate that flows through inner tube 288 is in turn maintained at a preset temperature.

Back plate 274 is further shown to include a bore 302 through which a proximity sensor 304 is matingly fitted. Sensor 304 could be considered the same as Bio-Test sensor 42 for determining whether or not the Temp Check calibration device 46 has been mated to housing plate 276. Proximity switch 304 is an electromagnetic switch that is activated when Temp Check connector 46, which is shaped similar to reflux connector 282, is properly mated to housing plate 276. A magnet embedded in the Temp Check connector 46 activates the proximity switch 304 to inform processor 26 that a test sequence for determining the integrity of the various components of the device of the instant invention is about to take place.

Back plate 274 moreover is shown to have another bore 304 through which a position switch 307, which is the same as disposable sensor 44 shown in Fig. 1, extends. Switch 307 is a conventional switch that includes a plunger 309; which, when reflux connector 28 is mated with housing plate 26 and correctly positioned therein, is forced down into the body of switch 206 to thereby signify to processor 26 that connector 282 is correctly positioned.

With reference to Figs. 11a-11c, housing plate 276 is shown with greater particularity. As illustrated, housing plate 276 has a main ring-shaped body 306 that has extending therefrom two oppositely located raised portions 308 and 310. Portion 308 has an extending lip 312 and a lead in guide slot or groove 314. Portion 310 likewise has an extending lip 316 with a lead in guide slot or groove 318. In the inner circumference of body 306, there is at least one indent 320 for accepting an extension 322 at a corresponding wing 324 of reflux connector 282. See Figs. 12 and 13. A stop 313 is provided at the end of each of grooves 314 and 318 to prevent further rotation of the base of reflux connector 282 with respect to housing plate 276.

Reflux connector 282, as best shown in Figs. 12, 13 and 14, has a base 326 that is substantially ring-shaped so as to be matable with housing plate 276. An upright portion 328 extends from base 326. Extending coplanarly from base 326 are wings 322a and 322b which are mated with portions 308 and 310 of housing plate 276, respectively. To be more specific, once reflux connector 282 is positioned or superposed over plate 276 with base 326 of connector 282 being in contact with body 306 of housing plate 276, by holding and then turning or rotating raised portion 328 of connector 282 coplanarly with respect to housing plate 276, wing 324a is led by means of guide groove 318 into raised portions 310 while wing 324b is led into raised portion 308 by means of guide groove 314. With wings 322a and 322b coming into contact and snugly fitting into their corresponding detents 320, a feedback is provided to the user via his or her fingers that indeed connector 282 is positioned correctly to housing plate 276. For additional feedback, a spear shaped arm 344 extending from base 326 provides a visual indication to the user that connector 282 is positioned properly with respect to housing plate 276.

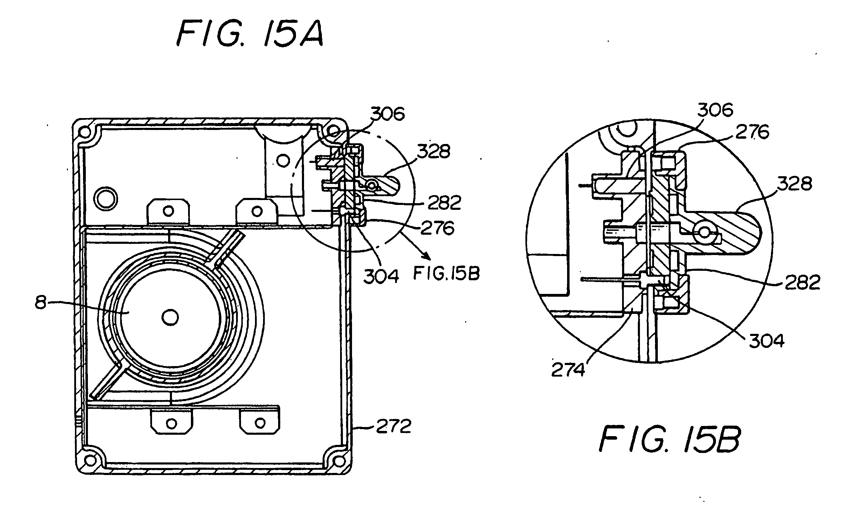

With connector 282 positioned properly with respect to housing plate 276, plunger 308 of switch 306 is pushed downwards to thereby generate a signal to processor 26 to inform the processor that indeed connector 282 has been fitted correctly to the connector assembly. If per chance base 326 of reflux connector 282 is not seated properly with respect to housing plate 276, an appropriate LED is lit at LED array 96 to inform the user that connector 282 has not been properly positioned onto the connector assembly, and the temperature regulation portion of the invention device accordingly will not be energized. See Figs. 15a and 15b for cross-sectional views illustrating the relationship of switch 306, the connector assembly and the reflux connector 282. As mentioned previously, Temp Check calibration device 46 is shaped the same as connector 282 with the exception that a magnet is embedded therein for activating proximity switch 304, when it is mated to housing plate 276.

Further with respect to Figs. 12, 13 and 14, note that connector 282 has a first opening 330 to which a fluid conduit such as for example 285 is fitted. Another fluid conduit such as for example 287 is fitted to an opening 332, which is located at the opposite side of upright portion 328. It is at opening 332 that the temperature regulated fluid flows for warming or cooling the infusate flowing through inner tube 288, which is connected to port 334.

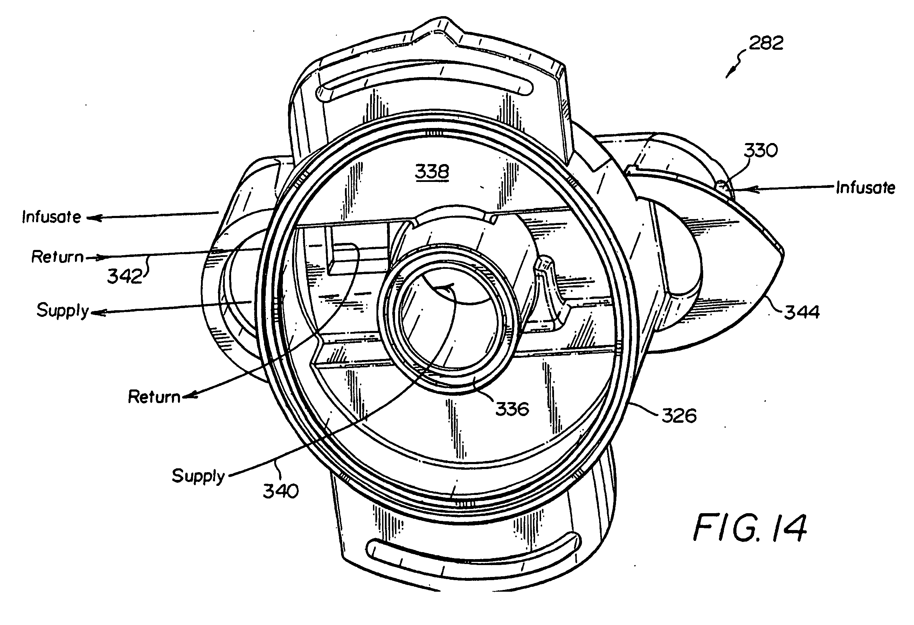

Fig. 14 illustrates in greater detail the respective flows of the infusate and the temperature regulating fluid to/from the fluid conduit via reflux connector 282. In particular, note that the bottom of base 326 has a center tubular port 336, which is aligned to and mated with supply port 298 when reflux connector 282 is properly mated to housing plate 276. The space defined between base 326 and central port 336 is an open space 338 that becomes fluidly connected to return port 300 when reflux connector 282 is properly positioned with respect to housing plate 276. Accordingly, with respect to reflux connector 282, the infusate is fed to the patient via opening 330 and then tube 288 as shown in Fig. 9. The supply fluid as indicated by supply line 340 is input from supply port 298 of back plate 274 to central port 336 of reflux connector 282 and then fed to outer tube 290 for warming or cooling the infusate. And the fluid is returned to heater/cooler 18 via return path 342 through space 338 to return port 30 of back plate 274.

In essence, to properly mate reflux connector 282 to housing plate 276, base 326 of reflux connector 282 has to be rotated coplanarly with respect to body 306 of housing plate 276, or flange 292 of back plate 274, until central port 336 of connector 282 is intimately aligned with supply port 298 of back plate 274 and opening 338 of connector 282 is in an unimpeded fluid communication path with return port 300. Gasket 278 of course provides the required sealing between port 336 and opening 338 of reflux connector 282 and supply port 298 and return port 300 of back plate 274, so that a closed loop fluid path between fluid conduit 284, at least with respect to outer tube 290 thereof, and the temperature regulating device of the instant invention is established. Ring gaskets, not shown, may also be provided between housing plate 276 and back plate 274, and between housing plate 276 and reflux connector 282, to prevent any leakage of fluid from the fluid path to the external environment.

Note further that return port 300 of back plate 274, once plate 274 is mounted to wall 272 of the housing of the device, is extended to an opening 301 (see Figs. 7 and 8) which in turn is connected to an extension port 303 mounted on top of a shroud 304, by means of a tubing, not shown. Extension port 303 is the return port to reservoir 8, which is covered by shroud 304.

When Temp Check calibration connector 46 is mated to housing plate 276 and is sensed by Bio-Test sensor 42, i.e., proximity sensor 304, a signal is provided to processor 26 to inform the system that self testing is to take place. With reference to the flow chart shown in Fig. 16, the self test, or bio-test, begins with step 346. With Temp Check 46 in place, the system waits for the activation of either on switch 104 per step 348, or off switch 106 per step 350. Once either on switch 104 or off switch 106 is pressed, the process proceeds to determine the level of testing to be done. A number of tests are then performed as shown in the flow chart of Fig. 16.

Fig. 17 is an illustration of the operation to be performed by the system to test the integrity of the various components. Again, it is based on Temp Check 46 being correctly mated to the connector assembly and thereafter the activation of the system by means of pressing on switch 104. The various states, from 0-9, relating to the self-testing operation are illustrated in the flow chart of Fig. 17.

Note that Temp Check connector 46 is structurally very similar to reflux connector 282 with the major differences being that no fluid conduits are connected thereto so that the fluid is refluxed directly in the Temp Check connector itself, and the embedding therein of a magnet for activating the bio-test sensor 42 when it is properly seated onto housing plate 276.

## Claims

1. A connector assembly (270) for coupling a heat exchange conduit (287) to a fluid temperature regulation unit (272), comprising: mounting means mounted at a given location to said fluid temperature regulation unit (272) to provide a communications path for a fluid whose temperature is regulated by said unit (272) to traverse out of and into said unit; and connector means (282) having coupled thereto said heat exchange conduit (287) matable with said mounting means (276), said connector means (282) having one port means (336) for receiving and routing said fluid from said fluid temperature regulation unit (272) to said heat exchange conduit (287) and another port means (338) for returning said fluid from said heat exchange conduit (287) to said fluid temperature regulation unit (272),
**characterised in that** said mounting means (276) is mounted coplanarly along one wall (272a) of said unit (272), said one and other port means (332) being configured in said connector means (282) to complete said communications path provided by said mounting means when said connector means (274) is demountably engaged to and sealingly mated with said mounting means by said connector means (282) being moved to a predetermined location coplanarly along said one wall (272a) relative to said mounting means (276).

2. A connector assembly according to claim 1, wherein said mounting means (276) comprises a plate (276) mounted to an outside surface (272a) of said fluid temperature regulation unit (272), said plate (276) including a pair of oppositely located raised portions (308,310) each with a lead in groove 314,318), an indent (320) being formed at the inner wall of each of said grooves 314,318); and wherein said connector means (282) comprises a base (326) having two wings (322a,322b) each for mating with a corresponding one of said raised portions (308,310), each of said wings (322a,322b) having an extension that fits into the indent (320) of said corresponding one raised portion (308,310) so that as said base (316) is rotated coplanarly along the plane of said plate (276), each of said wings (322a,322b) is guided into its corresponding raised portion (308,310) along its lead in groove (314,318), said base (326) being determined to be properly mated with said plate (276) when said respective extensions of said wings (322a,322b) are mated with the indents (320) of their corresponding raised portions (308,310).

3. A connector assembly according to claim 1 or claim 2, wherein said connector means (282) comprises a body (328) and said one port (336) means comprises a tubular bore internal of said body (328), said other port (338) means being formed by the space internal of said body (328) surrounding said tubular bore (336).

4. A connector assembly according to claim 1, wherein said fluid temperature regulation unit (272) includes a housing (272); and wherein said mounting means further comprises a back plate (274) mounted to the interior surface of said housing (272), said back plate (274) including a circular portion (284) mating with a hole formed on said housing (272) whereabout said back plate is mounted, a first conduit (298) extending through said circular portion to provide a path for said fluid which temperature is regulated by said fluid temperature regulation unit to output from said unit and a second conduit (300) extending through said base of said circular portion (284) to provide a path for said fluid to be returned to said fluid temperature regulation unit; and a housing plate (276) mounted to the exterior surface (272a) of said housing (272) and superposingly secured to said back plate (274), said housing plate (276) including a pair of oppositely located raised portions (302,210) each with a lead in groove (314,318).

5. A connector assembly according to claim 4, wherein said connector means (282) comprises a base (326) having two wings (322a,322b) each for mating with a corresponding one of said raised portions (308,310) of said housing plate (276), each of said wings (322a,322b) being rotatably guided into its corresponding raised portion by the rotation of said base (326) coplanarly relative to said housing plate (276), said base (326) being properly mated with said housing plate (276) when said base (326) can no longer be rotated relative to said housing plate (276).

6. A connector assembly according to any of the preceding claims, further comprising: switch means (307) positioned relative to said mounting means (276) for preventing said fluid temperature regulation unit from being energised when said connector means (232) is not positioned to said predetermined location relative to said mounting means (276).

7. A connector assembly according to any of the preceding claims, further comprising gasket means (278) interposed between said mounting means (276) and said connector means (282) to prevent leaks from said port means to thereby sealingly confine said fluid to said communications path.

8. A connector assembly according to claim 7, further comprising a second gasket means positioned at the outer circumferential periphery of said connector means (282) for preventing said fluid from escaping outside said connector assembly (270).

9. A connector assembly according to any of the preceding claims, further comprising sensor means (304) positioned relative to said mounting means (276) for informing said temperature regulation unit that calibration and temperature tests of various components therein are to take place.

10. A connector assembly according to any of the preceding claims, wherein said temperature regulation unit comprises a heat generator unit and said fluid comprises water being heated or cooled by said unit.

## Patentansprüche

1. Steckverbinder (270) zum Koppeln einer Wärmeaustauschleitung (287) an eine Fluidtemperaturreguliereinheit (272), der Folgendes umfasst: ein Befestigungsmittel, das an einer vorgegebenen Stelle an der Fluidtemperaturreguliereinheit (272) angebracht ist und für einen Verbindungsweg für ein Fluid, dessen Temperatur von der Einheit (272) reguliert wird, sorgt, damit dieses sich aus der Einheit heraus und in die Einheit hinein bewegt, und ein Verbindermittel (282), an das die Wärmeaustauschleitung (287) gekoppelt ist, die mit dem Befestigungsmittel (276) verbunden werden kann, wobei das Verbindermittel (282) ein Anschlussmittel (336) für das Aufnehmen und Leiten des Fluids aus der Fluidtemperaturreguliereinheit (272) zur Wärmeaustauschleitung (287) aufweist sowie ein weiteres Anschlussmittel (338) für das Rückführen des Fluids von der Wärmeaustauschleitung (287) zur Fluidtemperaturreguliereinheit (272),
**dadurch gekennzeichnet, dass** das Befestigungsmittel (276) koplanar an einer Wand (272a) der Einheit (272) entlang angebracht ist, wobei das eine und ein weiteres Anschlussmittel (332) bei dem Verbindermittel (282) so konfiguriert sind, dass sie den vom Befestigungsmittel bereitgestellten Verbindungsweg vervollständigen, wenn das Verbindermittel (274) abnehmbar in das Befestigungsmittel eingreift und dicht mit dem Befestigungsmittel verbunden ist, indem das Verbindermittel (282) in Bezug zu dem Befestigungsmittel (276) koplanar an der Wand (272a) entlang zu einer vorgegebenen Stelle bewegt wird.

2. Steckverbinder nach Anspruch 1, bei dem das Befestigungsmittel (276) eine Platte (276) umfasst, die an einer Außenfläche (272a) der Fluidtemperaturreguliereinheit (272) angebracht ist und ein Paar sich gegenüber liegende, erhabene Abschnitte (308, 310) aufweist, die jeweils mit einer Einlaufrille (314, 318) versehen sind, wobei an der Innenwand jeder Rille (314, 318) eine Kerbe (320) ausgebildet ist, und bei dem das Verbindermittel (282) einen Sockel (326) mit zwei Auskragungen (322a, 322b) umfasst, die jeweils mit einem entsprechenden erhabenen Abschnitt (308, 310) zusammenpassen und jeweils ein Ansatzstück aufweisen, das in die Kerbe (320) des entsprechenden erhabenen Abschnittes (308, 310) passt, so dass jede Auskragung (322a, 322b), wenn der Sockel (316) koplanar auf der Ebene der Platte (276) gedreht wird, an ihrer Einlaufrille (314, 318) entlang in ihren entsprechenden erhabenen Abschnitt (308, 310) geführt wird, wobei der Sockel (326) so bemessen ist, dass er ordnungsgemäß mit der Platte (276) verbunden ist, wenn die jeweiligen Ansatzstücke der Auskragungen (322a, 322b) mit den Kerben (320) ihrer entsprechenden erhabenen Abschnitte (308, 310) verbunden sind.

3. Steckverbinder nach Anspruch 1 oder Anspruch 2, bei dem das Verbindermittel (282) einen Körper (328) und das eine Anschlussmittel (336) eine in dem Körper (328) gelegene rohrförmige Bohrung umfasst, wobei das andere Anschlussmittel (338) durch den Raum in dem die rohrförmige Bohrung (336) umgebenden Körper (328) gebildet wird.

4. Steckverbinder nach Anspruch 1, bei dem zu der Fluidtemperaturreguliereinheit (272) ein Gehäuse (272) gehört und bei dem das Befestigungsmittel des Weiteren eine Montageplatte (274) umfasst, die an der Innenfläche des Gehäuses (272) angebracht ist und einen ringförmigen Abschnitt (284) aufweist, der mit einem am Gehäuse (272) ausgebildeten Loch zusammenpasst, um das herum die Montageplatte angebracht ist, wobei eine erste Leitung (298) durch den ringförmigen Abschnitt verläuft, einen Weg für das Fluid bereitstellt, dessen Temperatur von der Fluidtemperaturreguliereinheit reguliert wird, und es aus der Einheit herausleitet und eine zweite Leitung (300) durch den Sockel des ringförmigen Abschnittes (284) verläuft und einen Weg bereitstellt, auf dem das Fluid zur Fluidtemperaturreguliereinheit zurückgeführt wird, und eine Gehäuseplatte (276), die an der Außenfläche (272a) des Gehäuses (272) angebracht und die Montageplatte (274) überlagernd an dieser befestigt ist, wobei die Gehäuseplatte (276) ein Paar sich gegenüber liegende, erhabene Abschnitte (308, 310) umfasst, die jeweils mit einer Einlaufrille (314, 318) versehen sind.

5. Steckverbinder nach Anspruch 4, bei dem das Verbindermittel (282) einen Sockel (326) mit zwei Auskragungen (322a, 322b) umfasst, die jeweils mit einem entsprechenden erhabenen Abschnitt (308, 310) der Gehäuseplatte (276) zusammenpassen, wobei jede Auskragung (322a, 322b) durch das Drehen des Sockels (326) koplanar in Bezug zur Gehäuseplatte (276) drehend in ihren entsprechenden erhabenen Abschnitt geführt wird und der Sockel (326) ordnungsgemäß mit der Gehäuseplatte (276) verbunden ist, wenn er in Bezug zu dieser nicht mehr gedreht werden kann.

6. Steckverbinder nach einem der vorhergehenden Ansprüche, der des Weiteren Folgendes umfasst: in Bezug zum Befestigungsmittel (276) positionierte Schaltermittel (307), die verhindern, dass der Fluidtemperaturreguliereinheit Strom zugeführt wird, wenn das Verbindermittel (232) nicht an der vorgegebenen Stelle in Bezug zum Befestigungsmittel (276) positioniert ist.

7. Steckverbinder nach einem der vorhergehenden Ansprüche, der des Weiteren ein Dichtungsmittel (278) umfasst, das zwischen dem Befestigungsmittel (276) und dem Verbindermittel (282) angeordnet ist, um Leckagen am Anschlussmittel zu verhindern, und **dadurch** das Fluid auf dichte Weise auf den Verbindungsweg einschränkt.

8. Steckverbinder nach Anspruch 7, der des Weiteren ein zweites Dichtungsmittel umfasst, das am Außenumfang des Verbindermittels (282) positioniert ist und verhindert, dass das Fluid außerhalb des Steckverbinders (270) austreten kann.

9. Steckverbinder nach einem der vorhergehenden Ansprüche, der des Weiteren ein in Bezug zum Befestigungsmittel (276) positioniertes Sensormittel (304) umfasst, das die Temperaturreguliereinheit darüber informiert, dass Kalibrierung und Temperaturtests an verschiedenen darin befindlichen Komponenten stattfinden werden.

10. Steckverbinder nach einem der vorhergehenden Ansprüche, bei dem die Temperaturreguliereinheit eine Wärmeerzeugereinheit und das Fluid von der Einheit erwärmtes oder gekühltes Wasser umfasst.

## Revendications

1. Ensemble de connexion (270) pour raccorder un conduit échangeur de chaleur (287) à une unité de régulation de température de fluide (272) comprenant : des moyens de montage montés en un emplacement donné sur ladite unité de régulation de température de fluide (272) pour créer un chemin de communication pour un fluide dont la température est régulée par ladite unité (272) et qui traverse bidirectionnellement ladite unité; et des moyens de connexion (282) auxquels est connecté ledit conduit échangeur de chaleur (287), adaptable avec lesdits moyens de montage (276), lesdits moyens de connexion (282) ayant un moyen formant orifice (336) pour recevoir et acheminer ledit fluide à partir de ladite unité de régulation de température de fluide (272) jusqu'audit conduit échangeur de chaleur (287), et un autre moyen formant orifice (338) pour renvoyer ledit fluide à partir dudit conduit échangeur de chaleur (287) jusqu'à ladite unité de régulation de température de fluide (272),
**caractérisé en ce que** lesdits moyens de montage (276) sont coplanaires le long d'une paroi (272a) de ladite unité (272), l'un et l'autre des moyens (332) formant lesdits orifices étant tous deux configurés dans lesdits moyens de connexion (282) de sorte à compléter ledit chemin de communication créé par lesdits moyens de montage lorsque lesdits moyens de connexion (274) sont engagés de manière démontable et adaptés de façon étanche avec lesdits moyens de montage par déplacement coplanaire desdits moyens de connexion (282) le long de ladite paroi (272a) relativement auxdits moyens de montage (276), jusqu'à un emplacement prédéterminé.

2. Ensemble de connexion selon la revendication 1, dans lequel lesdits moyens de montage (276) comprennent une plaque (276) montée sur une surface extérieure (272a) de ladite unité de régulation de température de fluide (272), ladite plaque (276) incluant une paire de parties en relief mutuellement opposées (308, 310), chacune étant pourvue d'une rainure de guidage d'entrée (314, 318), un retrait (320) étant formé au niveau de la paroi intérieure de chacune desdites rainures (314, 318), et dans lequel lesdits moyens de connexion (282) comprennent une base (326) pourvue de deux oreilles (322a, 322b) qui s'adaptent chacune avec une partie correspondante desdites deux parties en relief (308, 310), chacune desdites oreilles (322a, 322b) ayant un prolongement qui s'adapte dans le retrait (320) de ladite partie en relief correspondante (308, 310) de sorte que, lorsque la base est en rotation coplanaire le long du plan de ladite plaque (276), chacune desdites oreilles (322a, 322b) est guidée dans sa partie en relief correspondante (308, 310) le long de sa rainure de guidage d'entrée (314, 318), ladite base (326) étant déterminée comme correctement adaptée avec ladite plaque (276) lorsque lesdits prolongements respectifs desdites oreilles (322a, 322b) sont adaptés avec les retraits (320) de leurs parties en relief correspondantes (308, 310).

3. Ensemble de connexion selon la revendication 1 ou la revendication 2, dans lequel lesdits moyens de connexion (282) comprennent un corps (328) et ledit premier moyen formant orifice (336) comprend un alésage tubulaire à l'intérieur dudit corps (328), ledit autre moyen formant orifice (338) étant formé par l'espace, à l'intérieur dudit corps (328), qui entoure ledit alésage tubulaire (336).

4. Ensemble de connexion selon la revendication 1, dans lequel ladite unité de régulation de température de fluide (272) comprend un boîtier (272); et dans lequel lesdits moyens de montage comprennent de plus une plaque d'appui (274) montée sur la surface intérieure dudit boîtier (272), ladite plaque d'appui (274) comportant une partie circulaire (284) qui s'adapte dans un trou formé sur ledit boîtier (272) là où ladite plaque d'appui est montée, un premier conduit (298) qui s'étend à travers ladite partie circulaire pour créer un chemin permettant audit fluide, dont la température est régulée, de sortir de ladite unité de régulation de température de fluide, et un second conduit (300) qui s'étend à travers ladite base de ladite partie circulaire (284) pour créer un chemin pour le retour dudit fluide dans ladite unité de régulation de température de fluide; et une plaque de logement (276) montée sur la surface extérieure (272a) dudit boîtier (272) et fixée en superposition sur ladite plaque d'appui (274), ladite plaque de logement (276) incluant une paire de parties en relief (302, 210) mutuellement opposées, chacune étant pourvue d'une rainure de guidage d'entrée (314, 318).

5. Ensemble de connexion selon la revendication 4, dans lequel lesdits moyens de connexion (282) comprennent une base (326) ayant deux oreilles (322a, 322b) dont chacune s'adapte avec l'une des parties en relief correspondantes (308, 310) de ladite plaque de logement (276), chacune desdites oreilles (322a, 322b) étant guidée de façon rotatoire dans la partie en relief qui lui correspond, par la rotation coplanaire de ladite base (326) relativement à ladite plaque de logement (276), ladite base (326) étant correctement adaptée avec ladite plaque de logement (276) lorsque ladite base (326) ne peut plus tourner relativement à ladite plaque de logement (276).

6. Ensemble de connexion selon l'une quelconque des revendications précédentes, qui comprend de plus : des moyens de commutation (307) positionnés relativement auxdits moyens de montage (276) de sorte à empêcher ladite unité de régulation de température de fluide d'être excitée lorsque lesdits moyens de connexion (232) ne se trouvent pas en ladite position prédéterminée relativement auxdits moyens de montage (276).

7. Ensemble de connexion selon l'une quelconque des revendications précédentes, qui comprend de plus un moyen formant joint étanche (278) interposé entre lesdits moyens de montage (276) et lesdits moyens de connexion (282) pour éviter les fuites depuis lesdits moyens formant orifice afin de retenir hermétiquement ledit fluide dans ledit chemin de communication.

8. Ensemble de connexion selon la revendication 7, qui comprend de plus un second moyen formant joint étanche positionné à la périphérie circonférentielle extérieure desdits moyens de connexion (282) pour empêcher ledit fluide de s'échapper hors dudit ensemble de connexion (270).

9. Ensemble de connexion selon l'une quelconque des revendications précédentes, qui comprend de plus des moyens formant capteur (304) positionnés relativement auxdits moyens de montage (276) pour signaler à ladite unité de régulation de température que des tests d'étalonnage et de température vont être réalisés au niveau de divers composants internes de ladite unité.

10. Ensemble de connexion selon l'une quelconque des revendications précédentes, dans lequel ladite unité de régulation de température comprend une unité génératrice de chaleur, et ledit fluide comprend de l'eau en cours de chauffage ou de refroidissement par ladite unité.
